# EUROPEAN PATENT APPLICATION

(11) **EP 4 763 960 A1**
(43) Date of publication of application: **24.06.2026**
(21) Application number: 24222770.0
(22) Date of filing: 23.12.2024
(51) Int. Cl.: C11B 9/00, A61Q 1/00, A61Q 5/00, A61Q 3/00, A61Q 13/00, A61Q 15/00, A61Q 19/00, A61Q 90/00, C11D 1/00, A61Q 17/00

(54) **ORGANIC COMPOUNDS**

(71) Applicant: Givaudan SA, 1214 Vernier (CH)
(72) Inventor: The designation of the inventor has not yet been filed
(74) Representative: Global Patents

(57) **Abstract**

There is provided a compound of formula (I) 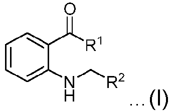 wherein
R¹ is methoxy, ethoxy, methyl or ethyl; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or bigger distance to N; or is selected from the group consisting of 3-methoxy-4-hydroxyphenyl and 3-ethoxy-4-hydroxyphenyl. The compound is able to release an alkyl anthranilate or (*o-*aminoalkylphenone and aldehyde R²-CHO.

## Description

### TECHNICAL FIELD

The present invention relates generally to fragrance precursors, which are able to release alkyl anthranilate and aldehyde. The invention also relates to perfume preparations and consumer products containing said precursors. The invention further relates to perfume preparations and consumer products, as well as the use of said perfume precursors and perfume preparations in consumer products, such as personal care and household care products.

### BACKGROUND

Perfumed consumer products such as cleaning or laundry products comprising fragrances are well-known in the art. However, it is known that fragrances can be altered when incorporated in certain consumer product bases, where alkalinity, acidity, the presence of oxidizing agents, such as hypochlorite salts, or other base components may lead to chemical degradation of the fragrance.

In addition, for some compounds like anthranilates o-aminoalkylphenone, discoloration issues in fragrance oils or detergent bases can occur. Methyl Anthranilate or o-aminoalkylphenones are key aroma constituents of many natural floral scents and fruit aromas and is very useful in fragrance composition. Thus, a color stable, efficient and biodegradable precursor of methyl anthranilate or o-aminoalkylphenones is highly sought after.

In JPH05140585 a group of anthranilates as fragrance compounds is described. However, the compounds are not biodegradable and their ability to release fragrant compounds is not described. There remains the need to provide a color stable, efficient and biodegradable precursor of alkyl anthranilate.

### SUMMARY

In accordance with a first aspect of the present invention there is provided a use of a compound of formula (I) as fragrance precursor 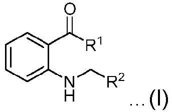 wherein
R¹ is methoxy, ethoxy, methyl or ethyl; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or farther from N; or is selected from the group consisting of 3-methoxy-4-hydroxyphenyl and 3-ethoxy-4-hydroxyphenyl.

In accordance with a second aspect of the present invention there is provided a compound of formula (I) as described above.

In accordance with a third aspect of the present invention there are provided fragrance compositions and consumer products comprising said compound of formula (I) as described above.

In accordance with a fourth aspect of the present invention there is provided a method to release fragrant compound from the compound of formula (I).

In accordance with a fifth aspect of the present invention there is provided a method of making said fragrance precursors, and fragrance compositions and consumer products comprising said fragrance precursors.

In accordance with a sixth aspect of the present invention there is provided a method to confer, enhance, improve or modify the hedonic properties of a fragrance composition or a consumer product by using the compound of formula (I).

Certain embodiments of any aspect of the present invention may provide one or more of the following advantages:
- release of anthranilates or o-aminoalkylphenones and aldehydes, wherein at least the anthranilate or the o-aminoalkylphenone is a fragrance compound;
- efficient delivery of the odors of anthranilates or o-aminoalkylphenones in consumer products;
- minimization of discoloration issues in comparison to accords using the free anthranilates; and
- provision of biodegradable fragrance precursors.

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based on the surprising finding that the compound of formula (I) is a biodegradable fragrance precursor able to release alkyl anthranilate or o-aminoalkylphenone and the corresponding aldehyde, which might be a fragrance aldehyde.

There is therefore provided herein the use of the compound of formula (I) as fragrance precursor 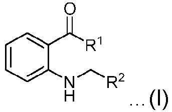 wherein
R¹ is methoxy, ethoxy, methyl or ethyl; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or farther from N; or is selected from the group consisting of 3-methoxy-4-hydroxyphenyl and 3-ethoxy-4-hydroxyphenyl.

The compound of formula (I) can release the corresponding (fragrance) aldehyde R²-CHO and methyl or ethyl anthranilate or o-aminoalkylphenones when exposed to air, thus leading to a long lasting fragrance impression, e.g. in consumer product applications. Furthermore, said compound is biodegradable.

Schiffbases (i.e. imines with the general motif C=NR^{X}, wherein R^{X} is not hydrogen) resulting from the condensation of alkyl anthranilate with fragrance aldehydes are known in the fragrance industry as fragrance precursors. Their disadvantage is that they are unstable and impart colorations. Reduction of the C=N double bond in Schiffbases to a C-N single bond (reduction of imine to amine) yields N-alkyl anthranilates, which would be expected to be odourless. The same holds for N-alkyl o-aminoalkylphenones. However, it was now surprisingly found that the compounds of formula (I), after exposure to ambient air, release alkyl anthranilates or o-aminoalkylphenones and the corresponding (fragrance) aldehyde by an unprecedented spontaneous oxidation process.

The released aldehyde R²-CHO can be a fragrance aldehyde, which provides an additional odor note to the released anthranilate or (*o*-aminoalkylphenone. Alternatively, the released aldehyde R²-CHO can be an aldehyde with a very weak odor, that does not alter the odor of the released anthranilate or o-aminoalkylphenones, which is then mainly determining the odor.

The biodegradability of the compound of formula (I) can be influenced by the nature of R². It was found that the compound is biodegradable if R² is derived from mainly linear fatty acid or terpenyl chains, optionally comprising a hydroxyl group in a certain distance. For example, the released hydroxy aldehyde is a 1,9-, a 1,10- or 1,11- hydroxyaldehyde. For example, the released aldehyde bears a phenyl ring at C(3) or in bigger distance to the carbonyl group. Alternatively, R² is derived from vanillin and ethyl vanillin.

The compound of formula (I) as defined above is biodegradable, in contrast to the structural closely related compounds described in JPH05140585, which are derived from heliotropin, anisic aldehyde, Cyclal C or Tropional.

For example, there is provided the use of the compound of formula (I) as fragrance precursor 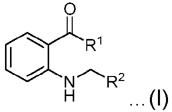 wherein
R¹ is methoxy or ethoxy; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or farther from N; or
is selected from the group consisting of 3-methoxy-4-hydroxyphenyl and 3-ethoxy-4-hydroxyphenyl.

For example, there is provided the use of the compound of formula (I) as fragrance precursor 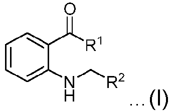 wherein
R¹ is methyl or ethyl; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or farther from N; or
is selected from the group consisting of 3-methoxy-4-hydroxyphenyl and 3-ethoxy-4-hydroxyphenyl.

For example, C6-C20 alkyl can be selected from the group consisting of hexyl, heptyl, octyl, nonyl, decyl, undecyl, dodecyl, tridecyl, tetradecyl, pentadecyl, hexadecyl, heptadecyl, octadecyl, nonadecyl, icosayl. For example, the alkyl is linear. Optionally, the alkyl chain can bear one or two methyl groups at any position, preferably not more than one methyl at each position. Optionally, the alkyl chain can contain one or two double bonds at any position, preferably isolated or conjugated double bonds. Optionally, the alkyl chain can contain a hydroxy group attached at C(8), C(9) or C(10),so that the released hydroxyaldehyde R²-CHO is a 1,9-, 1,10- or 1,11-hydroxyaldehyde. Optionally, the alkyl chain contains a phenyl ring attached at C(4) or in bigger distance to N. It should be noted that the R² can have none, or one, or more of the optional structural features like methyl group, double bond, phenyl group and /or hydroxyl group at the same time. For example, R² is a linear C6-C20 alkyl, that bears a phenyl group in at least C3 or farther from N, with optionally a double bond between phenyl and N and, optionally, a methyl group.

For example, there is provided the use of the compound of formula (I) as fragrance precursor, wherein the compound is selected from the group consisting of methyl 2-(decylamino)benzoate, methyl 2-(octylamino)benzoate, methyl 2-(heptylamino)benzoate, methyl 2-((2-methylundecyl)amino)benzoate, methyl 2-(undec-9-en-1-ylamino)benzoate, methyl (z)-2-(octadec-9-en-1-ylamino)benzoate, methyl 2-((9-hydroxy-5,9-dimethyldec-4-en-1-yl)amino)benzoate, methyl 2-((3-ethoxy-4-hydroxybenzyl)amino)benzoate, methyl 2-((4-hydroxy-3-methoxybenzyl)amino)benzoate, methyl 2-((3-methyl-5-phenylpentyl)amino)benzoate, methyl 2-(dodec-2-en-1-ylamino)benzoate, 1-(2-((3-methyl-5-phenylpentyl)amino)phenyl)ethan-1-one, ethyl 2-(decylamino)benzoate, ethyl 2-(octylamino)benzoate, ethyl 2-(heptylamino)benzoate, ethyl 2-((2-methylundecyl)amino)benzoate, ethyl 2-(undec-9-en-1-ylamino)benzoate, ethyl (Z)-2-(octadec-9-en-1-ylamino)benzoate, ethyl 2-((9-hydroxy-5,9-dimethyldec-4-en-1-yl)amino)benzoate, ethyl 2-((3-ethoxy-4-hydroxybenzyl)amino)benzoate, ethyl 2-((4-hydroxy-3-methoxybenzyl)amino)benzoate, ethyl 2-((3-methyl-5-phenylpentyl)amino)benzoate, ethyl 2-(dodec-2-en-1-ylamino)benzoate, and 1-(2-((3-methyl-5-phenylpentyl)amino)phenyl)propan- 1 -one.

Furthermore, there is provided the use of the compound of formula (I) as fragrance precursor, wherein the compound is selected from the group consisting of methyl 2-(dec-8-en-1-ylamino)benzoate, methyl 2-((2,6,10-trimethylundec-9-en-1-yl)amino)benzoate, ethyl 2-(dec-8-en-1-ylamino)benzoate and ethyl 2-((2,6,10-trimethylundec-9-en-1-yl)amino)benzoate.

For example, there is provided the use of the compound of formula (I) as fragrance precursor, wherein the compound is able to release a (fragrance) aldehyde R²-CHO, wherein R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or in bigger distance to the carbonyl group; or
is selected from the group consisting of 3-methoxy-4-hydroxyphenyl and 3-ethoxy-4-hydroxyphenyl.

For example, there is provided the use of the compound of formula (I) as fragrance precursor, wherein the compound is able to release a (fragrance) aldehyde R²-CHO, wherein R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or farther from the carbonyl group.

For example, there is provided the use of the compound of formula (I) as fragrance precursor, wherein the compound is able to release a (fragrance) aldehyde R²-CHO selected from the group consisting of 3-methoxy-4-hydroxyphenyl and 3-ethoxy-4-hydroxyphenyl.

For example, there is provided the use of the compound of formula (I) as fragrance precursor, wherein the compound is able to release a (fragrance) aldehyde R²-CHO selected from the group consisting of heptanal, octanal, decanal, 2-methylundecanal, citronellal, 6-Z-Nonenal, Citral, 2E,6Z-nonadienal, 5,9-dimethyl-9-hydroxydec-4-enal, 9-undecenal, 8-decenal, hexadecanal, undecanal, tetradecanal, oleyl aldehyde, ethyl vanillin, vanillin, 2-dodecanal 3-methyl dodecanal and 3-methyl-5-phenylpentanal.

For example, there is provided the use of the compound of formula (I) as fragrance precursor, wherein the compound is able to release a (fragrance) aldehyde R²-CHO and alkyl anthranilate or o-aminoalkylphenone, wherein the alkyl anthranilate or o-aminoalkylphenone ois selected from the group consisting of methyl 2-aminobenzoate, ethyl 2-aminobenzoate, 1-(2-aminophenyl)ethan-1-one and 1-(2-aminophenyl)propan-1-one.

The compound of formula (I) release alkyl anthranilate or o-aminoalkylphenone and the corresponding (fragrance) aldehyde upon exposure to ambient air by oxidation over a long period of time, e.g. one or several days such as 2-7 days or even longer.

Exposure of the compound of formula (I) to ambient air means exposure to molecular oxygen which might be responsible for the oxidative cleavage of the compound of formula (I) and the release of the alkyl anthranilate or o-aminoalkylphenone and the corresponding (fragrance) aldehyde The concentration of oxygen in the air is sufficient for cleaving the compound of formula (I) so that the cleavage products can be detected in the ambient air, e.g. by olfaction or GC-MS analysis of collected organic volatiles from the precursor headspace.

Some of the compounds are known from a different context than perfumery or fragrance, however, several compounds of formula (I) are novel. So in a further aspect of the present invention, there is provided a compound of formula (I) 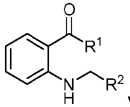 wherein
R¹ is methoxy, ethoxy, methyl or ethyl; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or bigger distance to N; or is selected from the group consisting of 3-methoxy-4-hydroxyphenyl and 3-ethoxy-4-hydroxyphenyl, with the proviso that the compound is not methyl 2-(octylamino)benzoate, methyl 2-(dodecylamino)benzoate, methyl 2-(hexadecylamino)benzoate or 1-(2-(octylamino)phenyl)ethan-1-one.

For example, the compound of formula (I) is selected from the group consisting of methyl 2-(decylamino)benzoate, methyl 2-(heptylamino)benzoate, methyl 2-((2-methylundecyl)amino)benzoate, methyl 2-(undec-9-en-1-ylamino)benzoate, methyl (z)-2-(octadec-9-en-1-ylamino)benzoate, methyl 2-((9-hydroxy-5,9-dimethyldec-4-en-1-yl)amino)benzoate, methyl 2-((3-ethoxy-4-hydroxybenzyl)amino)benzoate, methyl 2-((4-hydroxy-3-methoxybenzyl)amino)benzoate, methyl 2-((3-methyl-5-phenylpentyl)amino)benzoate, methyl 2-(dodec-2-en-1-ylamino)benzoate, 1-(2-((3-methyl-5-phenylpentyl)amino)phenyl)ethan-1-one, ethyl 2-(decylamino)benzoate, ethyl 2-(octylamino)benzoate, ethyl 2-(heptylamino)benzoate, ethyl 2-((2-methylundecyl)amino)benzoate, ethyl 2-(undec-9-en-1-ylamino)benzoate, ethyl (Z)-2-(octadec-9-en-1-ylamino)benzoate, ethyl 2-((9-hydroxy-5,9-dimethyldec-4-en-1-yl)amino)benzoate, ethyl 2-((3-ethoxy-4-hydroxybenzyl)amino)benzoate, ethyl 2-((4-hydroxy-3-methoxybenzyl)amino)benzoate, ethyl 2-((3-methyl-5-phenylpentyl)amino)benzoate, ethyl 2-(dodec-2-en-1-ylamino)benzoate, and 1-(2-((3-methyl-5-phenylpentyl)amino)phenyl)propan-1-one.

Furthermore, there is provided the the compound of formula (I) selected from the group consisting of methyl 2-(dec-8-en-1-ylamino)benzoate, methyl 2-((2,6,10-trimethylundec-9-en-1-yl)amino)benzoate, ethyl 2-(dec-8-en-1-ylamino)benzoate and ethyl 2-((2,6,10-trimethylundec-9-en-1-yl)amino)benzoate.

For example, there is provided the compound of formula (I), wherein the compound is able to release a (fragrance) aldehyde R²-CHO, wherein R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or in bigger distance to the carbonyl group; or
is selected from the group consisting of 3-methoxy-4-hydroxyphenyl and 3-ethoxy-4-hydroxyphenyl.

For example, there is provided the compound of formula (I), wherein the compound is able to release a (fragrance) aldehyde R²-CHO, wherein R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or in bigger distance to the carbonyl group.

For example, there is provided the compound of formula (I), wherein the compound is able to release a (fragrance) aldehyde R²-CHO selected from the group consisting of 3-methoxy-4-hydroxyphenyl and 3-ethoxy-4-hydroxyphenyl.

For example, there is provided the compound of formula (I), wherein the compound is able to release a (fragrance) aldehyde R²-CHO selected from the group consisting of heptanal, octanal, decanal, 2-methylundecanal, citronellal, 6-Z-Nonenal, Citral, 2E,6Z-nonadienal, 5,9-dimethyl-9-hydroxydec-4-enal, 9-undecenal, 8-decenal, hexadecanal, undecanal, tetradecanal, oleyl aldehyde, ethyl vanillin, vanillin, 2-dodecanal 3-methyl dodecanal and (3-methylhex-5-en-1-yl)benzene.

For example, there is provided the compound of formula (I), wherein the compound is able to release a (fragrance) aldehyde R²-CHO and alkyl anthranilate or o-aminoalkylphenone, wherein the alkyl anthranilate or o-aminoalkylphenone is selected from the group consisting of methyl 2-aminobenzoate, ethyl 2-aminobenzoate, 1-(2-aminophenyl)ethan-1-one and 1-(2-aminophenyl)propan-1-one.

The compounds of formula (I) are stable when their exposure to ambient air is limited or prevented, i.e. when stored in neat form in appropriate containers protected from air and light, or when stored in proper solvents, for example in ethanol, isopropanol, diethylene glycol monoethyl ether, glycerol, propylene glycol, 1,2-butylene glycol, dipropylene glycol, isopropyl myristate, pentane-1,2-diol, triethyl citrate, diethyl phthalate, triacetine and/or diacetine, or when incorporated in consumer products such as detergent, shampoo and fabric conditioner. Thus the compounds of formula (I) may find use in a broad range of consumer products in which a prolonged and defined release of fragrant compounds is desired.

In some embodiments, stabilizing compounds, for example alpha tocopherol, EDTA, ascorbic acid, BHT and related phenols, hydroquinones, Tinogard^{®} TT (BASF), Tinogard^{®} Q (BASF), can be added to the compounds of formula (I), for example in 0.01-1% by weight, to limit or prevent premature cleavage of the compound of formula (I) and the release of the compound of formula (II). In particular, the stabilizing compounds can be used to enhance the stability of the neat compounds of formula (I).

The compound of formula (I) may be used alone, or in combination with known odorant molecules selected from the extensive range of natural products, and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in perfume compositions, for example, carrier materials, and other auxiliary agents commonly used in the art.

In a further aspect, the compound of formula (I) may be used in combination with other fragrance precursors, either with further compounds of formula (I) or with precursors possessing a different chemical structure. A combination of precursors allows releasing a perfume accord, taking into account the changes over time.

In a further aspect, there is provided a fragrance composition comprising at least one compound of formula (I). For example, the fragrance composition is further comprising one or more known odourant molecules, and/or one or more ingredients or excipients conventionally used in conjunction with odourants in perfume compositions

As used herein, "carrier material" means a material which is practically neutral from a odourant point of view, i.e. a material that does not significantly alter the organoleptic properties of odourants.

The term "auxiliary agent" refers to ingredients that might be employed in a fragrance composition for reasons not specifically related to the olfactive performance of said composition. For example, an auxiliary agent may be an ingredient that acts as an aid to processing a fragrance ingredient or ingredients, or a composition containing said ingredient(s), or it may improve handling or storage of a fragrance ingredient or composition containing same. It might also be an ingredient that provides additional benefits such as imparting color or texture. It might also be an ingredient that imparts light resistance or chemical stability to one or more ingredients contained in a perfume composition. A detailed description of the nature and type of adjuvants commonly used in perfume compositions containing same cannot be exhaustive, but it has to be mentioned that said ingredients are well known to a person skilled in the art.

As used herein, "fragrance composition" means any composition comprising the compound of formula (I) and a base material, e.g. a solvent conventionally used in conjunction with odourants as listed above or an anti-oxidant adjuvant (stabilizing compound) as mentioned above.

The following non limiting list comprises examples of known odourant molecules, which may be combined with the compound of formula (I) in a fragrance composition:
- Essential oils and extracts, e.g. castoreum, costus root oil, oak moss absolute, geranium oil, tree moss absolute, basil oil, fruit oils, such as bergamot oil and mandarine oil, myrtle oil, palmarose oil, patchouli oil, petitgrain oil, jasmine oil, rose oil, sandalwood oil, wormwood oil, lavender oil and/ or ylang-ylang oil;
- Alcohols, e.g. cinnamic alcohol ((*E*)-3-phenylprop-2-en-1-ol); cis-3-hexenol ((Z)-hex-3-en-1-ol); Citronellol (3,7-dimethyloct-6-en-1-ol); dihydro myrcenol (2,6-dimethyloct-7-en-2-ol); Ebanol^{™} ((E)-3-methyl-5-(2,2,3-trimethylcyclopent-3-en-1-yl)pent-4-en-2-ol); eugenol (4-allyl-2-methoxyphenol); ethyl linalool ((E)-3,7-dimethylnona-1,6-dien-3-ol); farnesol ((2E,6Z)-3,7,11-trimethyldodeca-2,6,10-trien-1-ol); geraniol ((E)-3,7-dimethylocta-2,6-dien-1-ol); Super Muguet^{™} ((*E*)-6-ethyl-3-methyloct-6-en-1-ol); linalool (3,7-dimethylocta-1,6-dien-3-ol); menthol (2-isopropyl-5-methylcyclohexanol); Nerol (3,7-dimethyl-2,6-octadien-1-ol); phenyl ethyl alcohol (2-phenylethanol); Rhodinol^{™} (3,7-dimethyloct-6-en-1-ol); Sandalore^{™} (3-methyl-5-(2,2,3-trimethylcyclopent-3-en-1-yl)pentan-2-ol); terpineol (2-(4-methylcyclohex-3-en-1-yl)propan-2-ol); or Timberol^{™} (1-(2,2,6-trimethylcyclohexyl)hexan-3-ol); 2,4,7-trimethylocta-2,6-dien-1-ol, and/or [1-methyl-2(5-methylhex-4-en-2-yl)cyclopropyl]-methanol;
- Aldehydes and ketones, e.g. anisaldehyde (4-methoxybenzaldehyde); alpha amyl cinnamic aldehyde (2-benzylideneheptanal); Georgywood^{™} (1-(1,2,8,8-tetramethyl-1,2,3,4,5,6,7,8-octahydronaphthalen-2-yl)ethanone); Hydroxycitronellal (7-hydroxy-3,7-dimethyloctanal); Iso E Super^{®} (1-(2,3,8,8-tetramethyl-1,2,3,4,5,6,7,8-octahydronaphthalen-2-yl)ethanone); Isoraldeine^{®} ((E)-3-methyl-4-(2,6,6-trimethylcyclohex-2-en-1-yl)but-3-en-2-one); Hedione^{®} (methyl 3-oxo-2-pentylcyclopentaneacetate); Nympheal (3-(4-isobutyl-2-methylphenyl)propanal); Mahonial (5,9-dimethyl-9-hydroxy-decen-4-al); maltol; methyl cedryl ketone; methylionone; verbenone; and/or vanillin;
- Ether and acetals, e.g. Ambrox^{®} (3a,6,6,9a-tetramethyl-2,4,5,5a,7,8,9,9b-octahydro-1*H*-benzo[*e*][1]benzofuran); geranyl methyl ether ((2E)-1-methoxy-3,7-dimethylocta-2,6-diene); rose oxide (4-methyl-2-(2-methylprop-1-en-1-yl)tetrahydro-2/7-pyran); and/ or Spirambrene^{®} (2',2',3,7,7-pentamethylspiro[bicyclo[4.1.0]heptane-2,5'-[1,3]dioxane]);
- Esters and lactones, e.g. benzyl acetate; cedryl acetate ((1*S*,6*R*,8a*R*)-1,4,4,6-tetramethyloctahydro-1/7-5,8a-methanoazulen-6-yl acetate); γ-decalactone (6-pentyltetrahydro-2*H*-pyran-2-one); Helvetolide^{®} (2-(1-(3,3-dimethylcyclohexyl)ethoxy)-2-methylpropyl propionate); γ-undecalactone (5-heptyloxolan-2-one); and / or vetiveryl acetate ((4,8-dimethyl-2-propan-2-ylidene-3,3a,4,5,6,8a-hexahydro-1*H*-azulen-6-yl) acetate
- Macrocycles, e.g. Ambrettolide ((Z)-oxacycloheptadec-10-en-2-one); ethylene brassylate (1,4-dioxacycloheptadecane-5,17-dione); and / or Exaltolide^{®} (16-oxacyclohexadecan-1-one); and
- Heterocycles, e.g. isobutylquinoline (2-isobutylquinoline).

Overall, the compounds of formula (I) can be used alone, as a mixture thereof, or in combination with other fragrance ingredients and/or precursors thereof. Such other fragrance ingredients are also described, for example, in "Perfume and Flavor Chemicals", S. Arctander, Ed., Vol. I & II, Allured Publishing Corporation, Carol Stream, USA, 2003 and include fragrance compounds of natural or synthetic origin and essential oils.

In a further aspect, there is provided a consumer product comprising at least one compound of formula (I) and a consumer product base.

For example, the consumer product according to the invention is selected from the group consisting of detergents and cleaning agents, hygiene or care products, preferably in the field of body and hair care, cosmetics and household, preferably from the group consisting of perfume extracts, eau de parfums, eau de toilettes, aftershave lotions, eau de colognes, pre-shave products, splash colognes, perfumed refreshing wipes, acidic, alkaline or neutral detergents, textile fresheners, ironing aids, liquid detergents, powder detergents, laundry pretreatments, fabric softeners, laundry sheets, washing soaps, washing tablets, dish bar soaps, disinfectants, surface disinfectants, air fresheners, aerosol sprays, waxes and polishes, body care products, hand creams and lotions, foot creams and lotions, depilatory creams and lotions, after-shave creams and lotions, tanning creams and lotions, hair care products (in liquid or solid form), dry shampoo, deodorants, antiperspirants, decorative cosmetic products, candles, lamp oils, incense sticks, insecticides, repellents and fuels.

The consumer product for example is selected from fine fragrance, personal care products (body care products, hair care products, cosmetic products) fabric care products, home care products and air care products. As used herein, "consumer product base" means a composition for use as a consumer product to fulfill specific actions, such as cleaning, softening, and caring or the like.

Personal care products to which the compound of formula (I) can be added include for example all kinds of body care products. Especially interesting products are hair care products, for example shampoos, conditioners and hairsprays, and skin care products, like lotions or creams. Furthermore, the compound of formula (I) may be added to soaps, bath and shower gels and deodorants. The compound of formula (I) can be added to cosmetic products.

Home care products to which the compound of formula (I) can be added include all kinds of detergents, window cleaners, hard surface cleaners, all-purpose cleaners and furniture polishes. Preferably, the products are liquids, e.g. fabric detergent or conditioner compositions.

For example, the compounds of formula (I) can act as fragrance precursors in consumer products which further comprise enzymes.

The compound according to formula (I) may be used in a broad range of perfumed consumer products, e.g. in any field of fine and functional perfumery, such as perfumes, air care products, household products, laundry products, body care products and cosmetics. The compound can be employed in widely varying amounts, depending upon the specific article and on the nature and quantity of other odorant ingredients. The proportion of the formula (I) is typically from 0.0001 to 5 weight% of the article. In one embodiment, the compound of formula (I) may be employed in a fabric softener in an amount from 0.001 to 0.3 weight % (e.g. 0.01 to 0.1 including 0.05 weight%). In another embodiment, the compound of formula (I) may be used in fine perfumery but also in consumer products like shampoo, fabric softener or fabric detergents, in amounts from 0.001 to 30 weight% (e.g. up to about 10 or up to 20 weight%), more preferably between 0.01 and 5 weight%. However, these values are given only by way of example, since the experienced perfumer may also achieve effects or may create novel accords with lower or higher concentrations.

In one embodiment there is provided a consumer product comprising an acceptable amount of the compound of formula (I). For example, the fragranced article may comprise 0.000001 weight% to 90 weight% (including 0.00001 weight %; 0.0001 weight%, 0.001 weight%, 0.01 weight%, 0.05 weight%, 0.1 weight%, 0.5 weight%, 1 weight%, 5 weight%, 8 weight%, 10 weight%, 15 weight%, 20 weight%, 25 weight%, 30 weight%, 50 weight%, 60 weight%, 65 weight%) of the compound of formula (I) based on the total amount of the article.

The compound of formula (I) may be employed in a consumer product base simply by directly mixing the compound of the present invention, or a fragrance composition comprising the compound of formula (I), with the consumer product base, or it may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, and then mixed with the consumer product base. The consumer product base might further contain entrapment material able to release other fragrant compounds.

Thus, the invention additionally provides a method of manufacturing a consumer product, comprising the incorporation of a compound of formula (I) either by directly admixing it to the consumer product base or by admixing a fragrance composition comprising the compound of formula (I), which may then be mixed with a consumer product base, using conventional techniques and methods. Through the addition of an acceptable amount of the compound of the present invention as hereinabove described, the odor notes of an applied consumer product will be improved, conferred, enhanced, or modified.

Thus, the invention furthermore provides in another aspect a method to confer, enhance, improve or modify the hedonic properties of a fragrance composition or a consumer product, which method comprises adding to said composition or consumer product at least one compound of formula (I).

In general, the compounds of formula (I) may be prepared by reductive amination, i.e. the in situ condensation of, for example, methyl anthranilate and an aldehyde in a molar ratio of 1:1 to 1:2, in a suitable solvent such as methanol, in the presence of a hydrogenation catalyst such as palladium on activated charcoal, typically in amounts of 0.01-2 mol% Pd, under hydrogen, typically at a pressure of 10-20 bars, typically at room temperature. Alternatively, when the presence of a hydrogenation sensitive alkene in the aldehyde moiety precludes the application of catalytic hydrogenation, the reduction of the intermediate imine can be effected with a complex hydride, such as sodium cyanoborohydride, typically in equimolar or higher quantities, for example 2 molar equivalents. Alternatively, the N-alkylation of, for example, methyl anthranilate, can be effected with a suitable alkyl or substituted allyl or benzyl halide, such as 1-bromododec-2-ene, in the presence of a base, such as potassium carbonate, and, optionally, an additive such as potassium iodide, in a suitable solvent such as acetonitrile, at elevated temperature, for example 50-100°C, optionally in an autoclave which allows running the reaction at temperatures exceeding the boiling point of the employed solvent.

Yet another preparation method involves the hydrogenolysis of 2-alkyl-1,2-dihydro-4H-benzo[d][1,3]oxazin-4-ones, such as 2-nonyl-1,2-dihydro-4H-benzo[d][1,3]oxazin-4-one in the presence of hydrogen and a suitable catalyst, such as as palladium on activated charcoal, typically in amounts of 0.01-2 mol% Pd, under hydrogen, typically at a pressure of 1-10 bars, typically at room temperature, followed by esterification with methanol or ethanol. The esterification might be carried out in a one pot fasion by running the hydrogenolysis in the appropriate alcohol solvent, such as methanol, alternatively adding a catalyst, such as sulfuric acid.

In a further aspect, the compounds of the present invention are biodegradable, as demonstrated by the Manometric Respirometry test (OECD guideline for the testing of materials No. 301F, Paris 1992).

The invention is now further described with reference to the following non-limiting examples. These examples are for the purpose of illustration only and it is understood that variations and modifications can be made by one skilled in the art.

### EXAMPLES

### General:

All reactions were performed under argon using solvents and reagents from commercial suppliers without further purification. Solvents for extraction and chromatography were technical grade and used without further purification. Flash chromatography was performed using commercially available prepacked silica gel cartridges. Unless otherwise noted, a mixture of Heptane : MTBE was used as eluent. NMR spectra were recorded with Bruker AW 400 MHz or Avance III HD 400 MHz instruments. The chemical shifts for ¹H NMR spectra was reported in δ (ppm) referenced to the residual proton signal of the deuterated solvent; coupling constants were expressed in Hertz (Hz). ¹³C NMR spectra were referenced to the carbon signals of the deuterated solvent. The following abbreviations are used: s = singlet, d = doublet, t = triplet, q = quartet, quint. = quintuplet, m = multiplet, dd = double doublet, bs = broad singlet. GC/MS spectral data were obtained from an Agilent 6890 N and MSD 5975 using a column HP-5 MS, 30 m, 0.25 mm, 0.25 µm.

### Example 1: Methyl 2-(decylamino)benzoate

To the solution of methyl anthranilate (5.0 g, 33 mmol, 1.0 equiv) and decanal (7.8 g, 50 mmol, 1.5 equiv.) in methanol (50 mL) was added 5% palladium on activated charcoal (200 mg, 0.09 mmol Pd, 0.3 mol%). The mixture was placed in an autoclave vessel, pressurized with 10 bar hydrogen and stirred at room temperature for 20 hours. After pressure release and purging with nitrogen, MgSO₄ (9 g) was added and the mixture was filtered by suction. The filtrate was evaporated in a rotary evaporator to furnish a clear, yellow liquid (11.6 g), which was subjected to Kugelrohr distillation at 0.004 mbar/130-180°C to yield methyl 2-(decylamino)benzoate (5.0 g, 53%) as a clear yellow liquid.

Odour description (1% solution in EtOH on paper blotter, 24 h): green, floral.

¹H-NMR (500 MHz, CDCl₃) δ 7.90 - 7.95 (m, 1 H), 7.72 (br s, 1 H), 7.33 - 7.41 (m, 1 H), 6.70 (dd, *J*=8.5, 0.6 Hz, 1 H), 6.59 (ddd, *J*=8.0, 6.9, 1.2 Hz, 1 H), 3.88 (s, 3 H), 3.21 (td, *J*=7.0, 5.2 Hz, 2 H), 1.72 (dt, *J*=14.8, 7.2 Hz, 2 H), 1.42 - 1.54 (m, 2 H), 1.24 - 1.40 (m, 12 H), 0.93 (t, *J*=7.0 Hz, 3 H). ¹³C-NMR (126 MHz, CDCl₃) δ 169.1 (s), 151.3 (s), 134.6 (d), 131.6 (d), 114.1 (d), 111.2 (d), 51.4 (q), 42.9 (t), 31.9 (2t), 29.6 (t), 29.6 (t), 29.5 (t), 29.4 (t), 29.2 (t), 27.3 (t), 22.7 (t) 14.2 (t). GC/MS (El): *m*/*z* (%): 291 (9, M⁺), 260 (<1), 164 (73), 151 (5), 132 (100).

### Example 2: Methyl 2-(octylamino)benzoate

The protocol described in Example 1 was repeated with methyl anthranilate (5.0 g, 33 mmol, 1.0 equiv) and octanal (6.4 g, 50 mmol, 1.5 equiv.) in methanol (50 mL) to yield methyl 2-(octylamino)benzoate (5.30 g, 58%) as a slightly yellow liquid after bulb-to-bulb distillation (0.05 mbar/125-160°C).

Odour description (1% solution in EtOH on paper blotter, 24 h): orange blossom, sweet, fresh, bitter.

¹H-NMR (500 MHz, CDCl₃) δ 7.92 (dd, *J*=8.2, 1.5 Hz, 1 H), 7.71 (br. s, 1 H), 7.37 (ddd, *J*=8.5, 7.0, 1.8 Hz, 1 H), 6.68 - 6.72 (m, 1 H), 6.59 (ddd, *J*=8.0, 7.1, 1.1 Hz, 1 H), 3.88 (s, 3 H), 3.20 (td, *J*=7.0, 5.2 Hz, 2 H), 1.68 - 1.75 (m, 2 H), 1.43 - 1.50 (m, 2 H), 1.27 - 1.41 (m, 8 H), 0.92 (t, *J*=7.3 Hz, 3 H). ¹³C-NMR (126 MHz, CDCl₃) δ 169.1 (s), 151.3 (s), 134.6 (d), 131.6 (d), 114.1 (d), 111.2 (d), 109.6 (s), 51.4 (q), 42.9 (t), 31.9 (t), 29.4 (t), 29.3 (t), 29.2 (t), 27.3 (t), 22.7 (t), 14.1 (q). GC/MS (EI): *m*/*z* (%): 263 (9, M⁺), 232 (<1), 164 (59), 151 (4), 132 (100).

### Example 3: Methyl 2-(heptylamino)benzoate

The protocol described in Example 1 was repeated with methyl anthranilate (5.0 g, 33 mmol, 1.0 equiv) and heptanal (6.0 g, 50 mmol, 1.5 equiv.) in methanol (50 mL) to yield methyl 2-(heptylamino)benzoate (4.00 g, 50%) as a slightly yellow liquid after bulb-to-bulb distillation (0.08 mbar/120-150°C).

Odour description (1% solution in EtOH on paper blotter, 24 h): green, fatty, floral, orange blossom.

¹H-NMR (500 MHz, CDCl₃) δ 7.92 (dd, *J*=7.9, 1.5 Hz, 1 H), 7.71 (br s, 1 H), 7.37 (ddd, *J*=8.5, 7.0, 1.5 Hz, 1 H), 6.70 (dd, *J*=8.5, 0.6 Hz, 1 H), 6.59 (ddd, *J*=8.1, 7.0, 1.1 Hz, 1 H), 3.88 (s, 3 H), 3.20 (td, *J*=7.0, 5.2 Hz, 2 H), 1.72 (dt, *J*=14.8, 7.2 Hz, 2 H), 1.43 - 1.50 (m, 2 H), 1.28 - 1.41 (m, 6 H), 0.92 (t, *J*=7.0 Hz, 3 H).¹³C-NMR (126 MHz, CDCl₃) δ 169.1 (s), 151.3 (s), 134.6 (d), 131.6 (d), 114.1 (d), 111.2 (d), 109.6 (s), 51.4 (q), 42.9 (t), 31.8 (t), 29.2 (t), 29.1 (t), 27.2 (t), 22.7 (t), 14.1 (q). GC/MS (EI): *m*/*z* (%): 249 (8, M⁺), 216 (<1), 164 (52), 151 (3), 132 (100).

### Example 4: Methyl 2-((2-methylundecyl)amino)benzoate

The protocol described in Example 1 was repeated with methyl anthranilate (5.0 g, 33 mmol, 1.0 equiv) and 2-methyl undecanal (9.8 g, 53 mmol, 1.6 equiv.) in methanol (50 mL) to yield methyl 2-((2-methylundecyl)amino)benzoate (5.1 g, 48%) as a slightly yellow liquid after bulb-to-bulb distillation (0.04 mbar/130-160°C).

Odour description (1% solution in EtOH on paper blotter, 24 h): aldehydic, metallic.

¹H-NMR (500 MHz, CDCl₃) δ 7.92 (dd, *J*=8.2, 1.5 Hz, 1 H), 7.83 (br t, *J*=4.7 Hz, 1 H), 7.34 - 7.38 (m, 1 H), 6.69 (d, *J*=7.9 Hz, 1 H), 6.58 (ddd, *J*=8.1, 7.0, 1.1 Hz, 1 H), 3.88 (s, 3 H), 3.16 (dt, *J*=12.4, 5.6 Hz, 1 H), 3.00 (ddd, *J*=12.3, 7.2, 5.2 Hz, 1 H), 1.80 - 1.92 (m, 1 H), 1.21 - 1.53 (m, 17 H), 1.04 (d, *J*=6.7 Hz, 3 H), 0.91 (t, *J*=6.7 Hz, 3 H). ¹³C-NMR (126 MHz, CDCl₃) δ 169.2 (s), 151.5 (s), 134.6 (d), 131.7 (d), 114.0 (d), 111.2 (d), 109.5 (s), 51.4 (q), 49.2 (t), 34.8 (t), 32.8 (t), 31.9 (t), 29.9 (t), 29.7 (t), 29.7 (t), 29.4 (t), 27.0 (t), 22.7 (t), 18.2 (q), 14.1 (q). GC/MS (EI): *m*/*z* (%): 319 (6, M⁺), 288 (<1), 164 (95), 151 (3), 132 (100).

### Example 5: Methyl 2-(undec-9-en-1-ylamino)benzoate

The solution of methyl anthranilate (5.0 g, 33 mmol, 1 equiv.) and undec-9-enal (5.6 g, 33 mmol, 1 equiv.) in methanol (50 mL) was stirred for 3 days at room temperature. Then sodium cyanoborohydride (4.2 g, 66 mmol, 2 equiv.) was added in 3 portions, after which stirring was continued for 2 days. The following quenching and extraction was carried out in a well ventilated hood taking utmost to avoid exposure to vapours emanating from flasks ! The mixture was poured on ice-cold aqueous 2M HCl solution (30 mL), stirred for 10 min, then extracted with methyl t-butyl ether (100 mL). The organic layer was washed with water (100 mL) and diluted aqueous NaCl-solution (100 mL), then dried over MgSO₄. After filtration and concentration in a rotary evaporator an clear, orange-red liquid was obtained (8.9 g) which was bulb-to-bulb distilled to yield methyl 2-(undec-9-en-1-ylamino)benzoate (0.50 g, 5%) as a clear, yellow liquid (E/Z 2:1).

Odour description (1% solution in EtOH on paper blotter, 24 h): aldehydic.

¹H-NMR (500 MHz, CDCl₃) δ 7.92 (dd, *J*=8.2, 1.6 Hz, 1 H), 7.70 (br s, 1 H), 7.37 (ddd, *J*=8.6, 7.0, 1.7 Hz, 1 H), 6.69 (d, *J*=8.1 Hz, 1 H), 6.58 (ddd, *J*=8.0, 7.0, 1.1 Hz, 1 H), 5.39 - 5.49 (m, 2 H), 3.87 (s, 3 H), 3.17 - 3.23 (m, 2 H), 1.95 - 2.11 (m, 2 H), 1.69 - 1.75 (m, 1 H), 1.67 (dt, *J*=4.7, 1.3 Hz, 3 H), 1.55 - 1.64 (m, 1 H), 1.42 - 1.50 (m, 2 H), 1.27 - 1.41 (m, 8 H). ¹³C-NMR (126 MHz, CDCl₃, E-isomer) δ 169.1 (s), 151.3 (s), 134.6 (d), 131.6 (d), 124.6 (d), 123.6 (d), 114.1 (d), 111.2 (d), 109.6 (s), 51.4 (q), 42.9 (t), 32.6 (t), 29.6 (t), 29.4 (t), 29.4 (t), 29.2 (t), 29.1 (t), 27.2 (t), 17.9 (q). GC/MS (El): *m*/*z* (%): 303 (8, M⁺), 288 (<1), 164 (53), 151 (6), 132 (100).

### Example 6: Methyl (Z)-2-(octadec-9-en-1-ylamino)benzoate

The protocol described in Example 5 was repeated with methyl anthranilate (1.76 g, 11.6 mmol, 1.0 equiv), olealdehyde (3.10 g, 11.6 mmol, 1 equiv.) and cyano borohydride (1.46 g, 23.2 mmol, 2 equiv.) to yield methyl (Z)-2-(octadec-9-en-1-ylamino)benzoate (3.90 g, 84%) as a slightly yellow liquid after column chromatography on silica gel (heptane/MtBE 60:1 to 30:1).

Odour description (1% solution in EtOH on paper blotter, 24 h): orange blossom, green. ¹H-NMR (500 MHz, CDCl₃) δ 7.91 (dd, *J*=7.9, 1.6 Hz, 1 H), 7.70 (br s, 1 H), 7.37 (ddd, *J*=8.4, 7.1, 1.3 Hz, 1 H), 6.69 (d, *J*=8.6 Hz, 1 H), 6.58 (ddd, *J*=8.0, 7.0, 1.1 Hz, 1 H), 5.33 - 5.43 (m, 2 H), 3.87 (s, 3 H), 3.20 (td, *J*=7.0, 5.3 Hz, 2 H), 2.00 - 2.08 (m, 5 H), 1.66 - 1.76 (m, 2 H), 1.42 - 1.51 (m, 1 H), 1.23 - 1.39 (m, 20 H), 0.91 (t, *J*=7.1 Hz, 3 H). ¹³C-NMR (126 MHz, CDCl₃, E-isomer) δ 169.1 (s), 151.3 (s), 134.6 (d), 131.6 (d), 130.0 (d), 129.8 (d), 114.1 (d), 111.1 (d), 109.6 (s), 51.4 (q), 42.9 (t), 31.9 (t), 29.8 (2t), 29.5 (2t), 29.4 (t), 29.3 (4t), 29.2 (t), 27.2 (2t), 22.7 (t), 14.1 (q). GC/MS (EI): *m*/*z* (%): 401 (8, M⁺), 248 (<1), 164 (94), 151 (11), 132 (100).

### Example 7: Methyl 2-((9-hydroxy-5,9-dimethyldec-4-en-1-yl)amino)benzoate

The protocol described in Example 5 was repeated with methyl anthranilate (5.0 g, 33 mmol, 1.0 equiv), 9-hydroxy-5,9-dimethyldec-4-enal (6.6 g, 33 mmol, 1 equiv.) and cyano borohydride (4.2 g, 66 mmol, 2 equiv.) to yield methyl 2-((9-hydroxy-5,9-dimethyldec-4-en-1-yl)amino)benzoate (3.34 g, E/Z 74:25, 30%) as a slightly yellow liquid after bulb-to-bulb distillation (0.08 mbar/140-180°C).

Odour description (1% solution in EtOH on paper blotter, 24 h): aldehydic, sweet, floral.

¹H-NMR (500 MHz, CDCl₃, main E-diastereomer) δ 7.91 (dd, J=8.1, 1.7 Hz, 1 H), 7.71 (br s, 1 H), 7.36 (ddd, *J*=8.5, 7.0, 1.6 Hz, 1 H), 6.69 (d, *J*=8.6 Hz, 1 H), 6.53 - 6.62 (m, 1 H), 5.16 - 5.21 (m, 1 H), 3.86 (s, 3 H), 3.17 - 3.24 (m, 2 H), 2.16 (q, *J*=7.3 Hz, 2 H), 1.99 - 2.08 (m, 2 H), 1.69 - 1.81 (m, 4 H), 1.62 (s, 2 H), 1.42 - 1.49 (m, 4 H), 1.21 (d, *J*=7.1 Hz, 6 H). ¹³C-NMR (126 MHz, CDCl₃, E-isomer) δ 169.1 (s), 169.1 (s), 151.3 (s), 136.1 (s), 136.0 (s), 134.6 (d), 134.6 (d), 131.6 (d), 124.4 (d), 123.6 (d), 114.2 (d), 114.2 (d), 111.2 (d), 111.2 (d), 109.6 (s), 109.6 (s), 70.9 (s), 70.8 (s), 51.5 (q), 51.4 (q), 43.7 (t), 43.5 (t), 42.2 (t), 42.2 (t), 40.0 (t), 32.0 (t), 29.4 (t), 29.2 (2q), 29.2 (t), 25.4 (t), 23.3 (q), 22.6 (t), 22.6 (t), 15.9 (q). GC/MS (EI): *m*/*z* (%): 333 (5, M⁺), 318 (<1), 300 (<1), 190 (11), 177 (5), 164 (5), 151 (48), 132 (100).

### Example 8: Methyl 2-((3-ethoxy-4-hydroxybenzyl)amino)benzoate 8a) (4-(bromomethyl)-2-ethoxyphenoxy)(tert-butyl)dimethylsilane

The solution of ethyl vanillin (20.0 g, 120 mmol, 1 equiv.) and triethylamine (14.6 g, 144 mmol, 1.2 equiv.) in THF (150 mL) was cooled to 5°C and the solution of tert-butylchlorodimethylsilane (21.8 g, 144 mmol, 1.2 equiv.) in THF (80 mL) was added dropwise. The temperature rose to 10°C and the mixture turned yellow. Stirring was continued at room temperature for 2 h, then the mixture was poured on icecold saturated aqueous NH₄Cl solution. The mixture was extracted with MtBE (200 mL) and the organic layer was washed with water and brine and dried over MgSO₄. After filtration and removal of the solvents in a rotary evaporator, 4-((tert-butyldimethylsilyl)oxy)-3-ethoxybenzaldehyde was obtained as a clear yellow liquid (29.95 g, 89%). The crude product (107 mmol) was dissolved in methanol (250 mL) and the solution was cooled to 5°C, followed by the addition of NaBH₄ (3.23 g, 85.4 mmol, 0.8 equiv.) in 3 portions, upon which the temperature rose to 20°C. The mixture was stirred at room temperature for 1 h, then poured on ice-cooled 2M aqueous HCl-solution (250 mL). Extraction, drying and removal of the solvent was effected as described above to yield (4-((tert-butyldimethylsilyl)oxy)-3-ethoxyphenyl)methanol as a clear, colourless liquid (27.8 g, 92%). The crude product (98.4 mmol) was dissolved in hexane (250 mL), the solution was cooled to 5°C and the PBr₃ (8.79 g, 32.5 mmol, 0.33 equiv.) was added dropwise. The mixture was stirred for 1 h at room temperature, then poured onto ice-cooled 2M aqueous NaOH-solution (250 mL). Extraction, drying and removal of the solvent was effected as described above to yield (4-(bromomethyl)-2-ethoxyphenoxy)(tert-butyl)dimethylsilane as a colourless, clear liquid (28.9 g, 85%).

### 8b) Methyl 2-((3-ethoxy-4-hydroxybenzyl)amino)benzoate

Methyl anthranilate (7.22 g, 47.8 mmol, 1.1 equiv.) was dissolved in DMF (100 mL) and K₂CO₃ (6.60 g, 47.8 mmol, 1.1 equiv.) was added. The mixture was stirred at room temperature for 30 min, then above prepared (4-(bromomethyl)-2-ethoxyphenoxy)(tert-butyl)dimethylsilane (15.0 g, 43.4 mmol, 1 equiv.) was added dropwise. After completed addition, the mixture was heated to 80°C and stirred for 48 h. After cooling to room temperature, it was poured on water (100 mL). Extraction, drying and removal of the solvent was effected as described above in 7a) to yield methyl 2-((4-((tert-butyldimethylsilyl)oxy)-3-ethoxybenzyl)amino)benzoate as a clear yellow liquid (16.2 g, 90%), which was dissolved in THF (200 mL). After the addition of tetrabutylammonium fluoride (1 M in THF, 46.7 mL, 46.7 mmol, 1.2 equiv.), the mixture was stirred for 2 h, then poured on ice-cooled 2M aqueous HCl-solution (250 mL). Extraction, drying and removal of the solvent was effected as described above to yield a clear yellow liquid (12.14 g, >100%), which was purified by flash chromatography on silica gel (heptane/MTBE 2:1) to yield methyl 2-((3-ethoxy-4-hydroxybenzyl)amino)benzoate as a white solid (8.02 g, 68%).

Odour description (1% solution in EtOH on paper blotter, 24 h): soft, powdery, vanilla, orange blossom, marshmallow.

¹H-NMR (500 MHz, CDCl₃) δ 8.08 (br s, 1 H), 7.94 (dd, *J*=7.9, 1.3 Hz, 1 H), 7.88 (dd, *J*=8.1, 1.5 Hz, 1 H), 7.26 - 7.38 (m, 2 H), 6.68-6.93 (m, 1 H), 6.58 - 6.73 (m, 2 H), 5.62 (s, 1 H), 4.37 (d, *J*=5.4 Hz, 2 H), 4.11 (q, *J*=7.1 Hz, 2 H), 3.88 (s, 3 H), 1.44 (t, *J*=7.0 Hz, 3 H). ¹³C-NMR (126 MHz, CDCl₃) δ 169.1 (s), 151.0 (s), 146.0 (s), 144.9 (s), 134.6 (d), 131.6 (d), 130.6 (s), 120.0 (d), 114.8 (d), 114.4 (d), 111.7 (d), 110.6 (d), 110.1 (s), 64.5 (t), 51.5 (q), 47.0 (t), 14.1 (q). GC/MS (EI): *m*/*z* (%): 301 (19, M⁺), 286 (3), 268 (4), 166 (4), 151 (100), 123 (39).

### Example 9: Methyl 2-((4-hydroxy-3-methoxybenzyl)amino)benzoate

The preparation method described above in 7a-b) was repeated with vanillin and methyl anthranilate to yield methyl 2-((3-methoxy-4-hydroxybenzyl)amino)benzoate (6.53 g, yield of last step 60%) as a white solid.

Odour description (1% solution in EtOH on paper blotter, 24 h): soft, powdery, vanilla, orange blossom, marshmallow.

¹H-NMR (500 MHz, CDCl₃) δ 8.09 (br s, 1 H), 7.94 (dd, *J*=7.6, 1.7 Hz, 1 H), 7.34 (ddd, *J*=8.7, 6.9, 1.7 Hz, 1 H), 6.90 (s, 1 H), 6.89 (s, 2 H), 6.67 - 6.70 (m, 1 H), 6.63 (ddd, *J*=8.0, 7.0, 1.1 Hz, 1 H), 4.38 (d, *J*=5.4 Hz, 2 H), 3.89 (s, 3 H), 3.88 (s, 3 H), 1.58 (s, 1 H).

¹³C-NMR (126 MHz, CDCl₃) δ 169.1 (s), 151.0 (s), 146.7 (s), 144.8 (s), 134.6 (d), 131.6 (d), 130.7 (s), 120.1 (d), 114.9 (d), 114.4 (d), 111.7 (d), 110.2 (s), 109.7 (d), 55.9 (q), 51.5 (q), 47.0 (t). GC/MS (El): *m*/*z* (%): 287 (32, M⁺), 272 (4), 254 (9), 151 (100), 137 (98), 119 (55).

### Example 10: Methyl 2-((3-methyl-5-phenylpentyl)amino)benzoate

The protocol described in Example 1 was repeated with methyl anthranilate (5.0 g, 33 mmol, 1.0 equiv) and 3-methyl-5-phenylpentanal (8.7 g, 50 mmol, 1.5 equiv.) in methanol (50 mL) to yield methyl 2-((3-methyl-5-phenylpentyl)amino)benzoate (5.8 g, 56%) as a slightly yellow liquid after flash chromatography on SiO₂ (heptane/MTBE 9:1) and removal of residual starting material in a Kugelrohr oven (0.07 mbar/120°C).

Odour description (1% solution in EtOH on paper blotter, 24 h): orange blossom, citrus.

¹H-NMR (500 MHz, CDCl₃) δ 7.96 (dd, *J*=8.1, 1.7 Hz, 1 H), 7.72 (br s, 1 H), 7.41 (ddd, *J*=8.5, 7.0, 1.6 Hz, 1 H), 7.31 - 7.36 (m, 2 H), 7.24 (d, *J*=7.1 Hz, 3 H), 6.73 (d, *J*=8.3 Hz, 1 H), 6.60 - 6.65 (m, 1 H), 3.90 (s, 3 H), 3.19 - 3.35 (m, 2 H), 2.62 - 2.79 (m, 2 H), 1.69 - 1.91 (m, 3 H), 1.55 - 1.67 (m, 2 H), 1.08 (d, *J*=6.4 Hz, 3 H). ¹³C-NMR (126 MHz, CDCl₃) δ 169.1 (s), 151.3 (s), 142.8 (s), 134.6 (d), 131.7 (d), 128.4 (2d), 128.4 (d), 128.3 (d), 125.7 (d), 114.2 (d), 111.2 (d), 109.7 (s), 51.4 (q), 40.8 (t), 39.0 (t), 36.2 (t), 33.4 (t), 30.6 (d), 19.6 (q). GC/MS (EI): *m*/*z* (%): 311 (13, M⁺), 164 (59), 151 (16), 132 (100), 105 (6), 91 (26).

### Example 11: 1-(2-((3-methyl-5-phenylpentyl)amino)phenyl)ethan-1-one

The protocol described in Example 1 was repeated with 1-(2-aminophenyl)ethan-1-one (5.0 g, 37 mmol, 1.0 equiv) and 3-methyl-5-phenylpentanal (9.8 g, 55 mmol, 1.5 equiv.) in methanol (50 mL) to yield 1-(2-((3-methyl-5-phenylpentyl)amino)phenyl)ethan-1-one (4.3 g, 39%) as a yellow liquid after flash chromatography on SiO₂ (heptane/MTBE, gradient 19:1 to 9:1) and removal of residual starting material in a Kugelrohr oven (0.07 mbar/100°C).

Odour description (1% solution in EtOH on paper blotter, 24 h): linden blossom, orange blossom, citronella, grapes, strawberry, elderflower.

¹H-NMR (500 MHz, CDCl₃) δ 8.92 (br s, 1 H), 7.79 (dd, *J*=8.1, 1.7 Hz, 1 H), 7.40 (ddd, *J*=8.6, 7.1, 1.5 Hz, 1 H), 7.30 - 7.36 (m, 2 H), 7.24 (d, *J*=7.3 Hz, 3 H), 6.75 (d, *J*=8.6 Hz, 1 H), 6.59 - 6.66 (m, 1 H), 3.20 - 3.36 (m, 2 H), 2.64 - 2.81 (m, 2 H), 2.62 (s, 3 H), 1.53 - 1.93 (m, 5 H), 1.08 (d, *J*=6.4 Hz, 3 H). ¹³C-NMR (126 MHz, CDCl₃) δ 200.7 (s), 151.2 (s), 142.8 (s), 135.1 (d), 132.8 (d), 128.4 (2d), 128.4 (2d), 125.7 (d), 117.4 (d), 113.7 (d), 111.6 (s), 40.6 (t), 39.0 (t), 36.1 (t), 33.4 (t), 30.6 (q), 27.9 (d), 19.6 (q). GC/MS (EI): *m*/*z* (%): 295 (20, M⁺), 148 (100), 135 (30), 130 (44), 120 (13), 106 (13), 91 (48).

### Example 12: Methyl (E)-2-(dodec-2-en-1-ylamino)benzoate

The solution of methyl anthranilate (5.0 g, 33 mmol, 1.0 equiv) and (E)-1-bromododec-2-ene (9.0 g, 36 mmol, 1.1 equiv.) in acetonitrile (50 mL) was placed in an autoclave. After the addition of potassium carbonate (9.1 g, 66 mmol, 2 equiv.) and potassium iodide (5.5 g, 33 mmol, 1 equiv.), the autoclave was closed and the mixture was stirred at 90°C for 2 days. After cooling to room temperature, the mixture was poured on ice-cold water (150 mL), followed by extraction with MtBE (100 mL). The organic layer was washed with water (100 mL) and brine (100 mL). The dark brown liquid (9.34 g) obtained after drying on MgSO₄ and concentration in a rotary evaporator was purified by flash chromatography on SiO₂ (heptane/MTBE, gradient 30:1 to 86:14) to yield methyl (E)-2-(dodec-2-en-1-ylamino)benzoate (5.2 g, 50%) as a clear yellow liquid.

Odour description (1% solution in EtOH on paper blotter, 24 h): aldehydic, mandarin.

¹H-NMR (500 MHz, CDCl₃) δ 7.93 (dd, *J*=7.9, 1.6 Hz, 1 H), 7.79 (br s, 1 H), 7.36 (ddd, J=8.6, 7.0, 1.7 Hz, 1 H), 6.68 - 6.73 (m, 1 H), 6.60 (ddd, *J*=8.0, 7.0, 1.1 Hz, 1 H), 5.74 (dtt, *J*=15.2, 6.7, 6.7, 1.4, 1.4 Hz, 1 H), 5.53 - 5.63 (m, 1 H), 3.88 (s, 3 H), 3.68-3.85 (m, 2 H), 1.99 - 2.12 (m, 2 H), 1.23-1.52 (m, 14 H), 0.92 (t, *J*=6.6 Hz, 3 H). ¹³C-NMR (126 MHz, CDCl₃) δ 169.1 (s), 151.0 (s), 134.5 (d), 133.3 (d), 131.6 (d), 125.9 (d), 114.4 (d), 111.5 (d), 109.9 (s), 51.4 (q), 44.8 (t), 29.6 (t), 29.5 (2t), 29.4 (2t), 29.2 (t), 29.2 (t), 22.7 (t), 14.1 (q). GC/MS (EI): *m*/*z* (%): 317 (16, M⁺), 302 (5), 284 (3), 190 (23), 182 (7), 151 (100), 132 (44), 119 (33).

### Example 13: Application test

Compounds of the present invention, methyl 2-(octylamino)benzoate (Compound of Example 2), methyl 2-((3-methyl-5-phenylpentyl)amino)benzoate (Compound of Example 10) and 1-(2-((3-methyl-5-phenylpentyl)amino)phenyl)ethan-1-one (Compound of Example 11) were incorporated at 0.2% by weight into unperfumed liquid detergent base by magnetic stirring at room temperature for 24 h. A 40°C machine wash cycle was performed using 55 g of the above prepared liquid detergent and odour-neutral cotton/elastan mixed fabric T-shirts. The wet and line-dried fabric (1, 3 and 7 days) was assessed by a panel of 4-6 experts with regard to odour intensity and quality. The odour intensity was recorded on an intensity scale of 0 (odourless) to 5 (extremely strong). As can be seen from the table below, the fabric washed with compounds of the present invention exhibited a long lasting fresh orange blossom and green scent with additional floral and fruity facettes difficult to achieve otherwise through fragranced detergent application.

| Compound of example | wet | 1 day | 3 days | 7 days | odour on dry fabric |
|---|---|---|---|---|---|
| 2 | 2.0 | 2.2 | 1.8 | 2.3 | orange blossom, green, sweet, rosy, fresh |
| 10 | 2.4 | 2.7 | 2.8 | n.d. | orange blossom, hawthorn, citrus, soap, sweet |
| 11 | 2.9 | 2.9 | 3.0 | n.d. | orange blossom, powdery, anisic, citrus, soap |

### Example 14: Headspace analysis of released volatiles

A solution of the compound of formula (I) (100 µg, 1.0 mg/mL solution in MTBE) was evenly applied through a displacement pipette to a paper strip (1 cm x 8 cm). The paper strip was left to dry (1 h at room temperature), then folded in the middle and inserted in "V"-shape into a 20 mL headspace vial (Figure 3). The vial was closed with a septum screwcap and placed in a cabinet equipped with a fluorescent daylight imitation lamp and mirrored walls for 24 h (temperature 27-28°C).

Extraction and analysis of the released organic volatiles was effected in automated mode on a Trace 1310 gas chromatograph equipped with a TriPlus RSH autosampler (Thermo Fisher Scientific), coupled to a ISQ LT mass spectrometer (Thermo Fisher Scientific). The extraction of the organic volatiles from the headspace with SPME (solid phase microextraction) was carried out during 1 h at 40°C using a 50/30µm divinylbenzene/carboxen/ polydimethylsiloxane SPME fibre (DVB/CAR/PDMS, Supelco PIN 57298-U), and subsequently desorbed in splitless mode at 250°C for 1 min. GC-MS analysis (gas chromatography with mass spectrometric detection) was carried out with a VF-WAXms capillary column (Agilent, 30m length, 0.25mm I.D., 0.25µm film thickness). Helium was used as a carrier gas with a constant flow rate of 1ml/min. The GC oven temperature was programmed from 35°C with 2-min hold to 250°C at 5°C/min with a 10-min final temperature hold. The mass spectrometer was operated at 70 eV in EI mode over a m/z range of 33-350, scanned at 0.2 s intervals and with a temperature of transfer line and ion source of 230 °C and 220°C, respectively. The main sensorially active volatiles released from the compounds of formula (I) are reported in the Table below (indicated are relative peak area %; in bold: main odor vectors).

**Table 4**

| **Precursor** | **Released Volatiles** |
|---|---|
| Methyl 2-(octylamino)benzoate (Example 2) | **octanal** (76%), **methyl anthranilate** (9.5%), methyl 2-(octylideneamino)benzoate (1.4%, broad GC-peak). |
| Methyl 2-(decylamino)benzoate (Example 1) | **decanal** (69%), **methyl anthranilate** (9.1%), methyl 2-(decylideneamino)benzoate (2.5%, broad GC-peak). |
| Methyl 2-((2-methylundecyl) amino) benzoate (Example 4) | 2-undecanone (30%), **2-methylundecanal** (43%), **methyl anthranilate** (5.0%), methyl 2-((2-methylundecylidene)amino)benzoate (0.5%). |

It should be noted that the indicated relative peak area % values are not related to molar release rates. The relative amount of each volatile in the gas phase depends mainly on volatility.

### Example 15: Assessment of biodegradability

The results of the biodegradability assessment by the manometric respirometry test (OECD guideline for the testing of materials No. 301F, Paris 1992) are summarized in Table 5.

| Compound of example | Compound name | Biodegradation in % | result |
|---|---|---|---|
| 3 | methyl 2-(heptylamino)benzoate | 74 | inherently biodegradable |
| 2 | methyl 2-(octylamino)benzoate | 82 | inherently biodegradable |
| 1 | methyl 2-(decylamino)benzoate | 79 | inherently biodegradable |
| 7 | methyl 2-((9-hydroxy-5,9-dimethyldec-4-en-1-yl)amino)benzoate | 77 | inherently biodegradable |
| 8 | methyl 2-((3-ethoxy-4-hydroxybenzyl)amino)benzoate | 75 | readily biodegradable |
| 9 | methyl 2-((3-methoxy-4-hydroxybenzyl)amino)benzoate | 91 | readily biodegradable |
| 10 | Methyl 2-((3-methyl-5-phenylpentyl)amino)benzoate | 65 | inherently biodegradable |
| 11 | 1-(2-((3-methyl-5-phenylpentyl)amino)phenyl)ethan-1-one | 78 | inherently biodegradable |
| 12 | Methyl (*E*)-2-(dodec-2-en-1-ylamino)benzoate | 63 | inherently biodegradable |

The results show that compounds of the present invention with varying phenol moieties and side chains are biodegradable. A compound can be classified biodegradable, if it reaches the pass level of 60% oxygen consumption of theory required for complete mineralization.

A compound is readily biodegradable, if the pass level is reached within 10 days within the 28-day period of the test. The 10-day window begins when the degree of biodegradation has reached 10%. If the pass level is obtained after 28-day period of the test, the compound can be classified as inherently biodegradable.

For the sake of comparison, counterexamples of JPH05140585 have been prepared according to the indicated procedure in the patent and submitted to the above described biodegradability test. All compounds were not inherently biodegradable after 60 days as shown in the below table.

| Compound name | Biodegradation in % | result |
|---|---|---|
| methyl 2-(((2,4-dimethylcyclohex-3-en-1- | 8 | NOT inherently biodegradable |
| yl)methyl)amino)benzoate | | |
| methyl 2-((7-hydroxy-3,7-dimethyloctyl)amino)benzoate | 19 | NOT inherently biodegradable |
| methyl 2-((benzo[d][1,3]dioxol-5-ylmethyl)amino)benzoate | 13 | NOT inherently biodegradable |

## Claims

1. Use of the compound of formula (I) as fragrance precursor 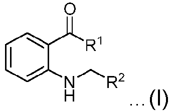 wherein
R¹ is methoxy, ethoxy, methyl or ethyl; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(3) or bigger distance to N; or is selected from the group consisting of 3-methoxy-4-hydroxyphenyl and 3-ethoxy-4-hydroxyphenyl.

2. The use according to claim 1, wherein the compound of formula (I) is selected from the group consisting of methyl 2-(decylamino)benzoate, methyl 2-(octylamino)benzoate, methyl 2-(heptylamino)benzoate, methyl 2-((2-methylundecyl)amino)benzoate, methyl 2-(undec-9-en-1-ylamino)benzoate, methyl (z)-2-(octadec-9-en-1-ylamino)benzoate, methyl 2-((9-hydroxy-5,9-dimethyldec-4-en-1-yl)amino)benzoate, methyl 2-((3-ethoxy-4-hydroxybenzyl)amino)benzoate, methyl 2-((4-hydroxy-3-methoxybenzyl)amino)benzoate, methyl 2-((3-methyl-5-phenylpentyl)amino)benzoate, methyl 2-(dodec-2-en-1-ylamino)benzoate, 1-(2-((3-methyl-5-phenylpentyl)amino)phenyl)ethan-1-one, ethyl 2-(decylamino)benzoate, ethyl 2-(octylamino)benzoate, ethyl 2-(heptylamino)benzoate, ethyl 2-((2-methylundecyl)amino)benzoate, ethyl 2-(undec-9-en-1-ylamino)benzoate, ethyl (Z)-2-(octadec-9-en-1-ylamino)benzoate, ethyl 2-((9-hydroxy-5,9-dimethyldec-4-en-1-yl)amino)benzoate, ethyl 2-((3-ethoxy-4-hydroxybenzyl)amino)benzoate, ethyl 2-((4-hydroxy-3-methoxybenzyl)amino)benzoate, ethyl 2-((3-methyl-5-phenylpentyl)amino)benzoate, ethyl 2-(dodec-2-en-1-ylamino)benzoate, and 1-(2-((3-methyl-5-phenylpentyl)amino)phenyl)propan-1-one.

3. A compound of formula (I) 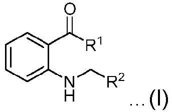 wherein
R¹ is methoxy, ethoxy, methyl or ethyl; and
R² is C6-C20 alkyl, optionally containing one or two double bonds, and optionally containing one or two methyl groups, and optionally containing a hydroxy group attached at C(8), C(9) or C(10), and optionally containing a phenyl ring attached at C(4) or bigger distance to N; or is selected from the group consisting of 3-methoxy-4-hydroxyphenyl and 3-ethoxy-4-hydroxyphenyl, with the proviso that the compound is not methyl 2-(octylamino)benzoate, methyl 2-(dodecylamino)benzoate, methyl 2-(hexadecylamino)benzoate or 1-(2-(octylamino)phenyl)ethan-1-one.

4. The compound of formula (I) selected from the group consisting of methyl 2-(decylamino)benzoate, methyl 2-(heptylamino)benzoate, methyl 2-((2-methylundecyl)amino)benzoate, methyl 2-(undec-9-en-1-ylamino)benzoate, methyl (z)-2-(octadec-9-en-1-ylamino)benzoate, methyl 2-((9-hydroxy-5,9-dimethyldec-4-en-1-yl)amino)benzoate, methyl 2-((3-ethoxy-4-hydroxybenzyl)amino)benzoate, methyl 2-((4-hydroxy-3-methoxybenzyl)amino)benzoate, methyl 2-((3-methyl-5-phenylpentyl)amino)benzoate, methyl 2-(dodec-2-en-1-ylamino)benzoate, 1-(2-((3-methyl-5-phenylpentyl)amino)phenyl)ethan-1-one, ethyl 2-(decylamino)benzoate, ethyl 2-(octylamino)benzoate, ethyl 2-(heptylamino)benzoate, ethyl 2-((2-methylundecyl)amino)benzoate, ethyl 2-(undec-9-en-1-ylamino)benzoate, ethyl (Z)-2-(octadec-9-en-1-ylamino)benzoate, ethyl 2-((9-hydroxy-5,9-dimethyldec-4-en-1-yl)amino)benzoate, ethyl 2-((3-ethoxy-4-hydroxybenzyl)amino)benzoate, ethyl 2-((4-hydroxy-3-methoxybenzyl)amino)benzoate, ethyl 2-((3-methyl-5-phenylpentyl)amino)benzoate, ethyl 2-(dodec-2-en-1-ylamino)benzoate, and 1-(2-((3-methyl-5-phenylpentyl)amino)phenyl)propan-1-one.

5. A fragrance composition comprising at least one compound of formula (I) as defined in claim 1.

6. A consumer product comprising at least one compound of formula (I) as defined in claim 1 and a consumer product base.

7. A method to release one or more fragrant compounds wherein a compound of formula (I) as defined in claim 1 is exposed to ambient air.

8. The method according to claim 7, wherein the released one or more fragrant compound is methyl or ethyl anthranilate and aldehyde R²-CHO.

9. A method to confer, enhance, improve or modify the hedonic properties of a fragrance composition or a consumer product, which method comprises adding to said composition or consumer product at least one compound of formula (I) as defined in claim 1.
